(19) Europäisches Patentamt
European Patent Office
Office européen des brevets

(11) Numéro de publication : **0 347 305 B1**

# (12) FASCICULE DE BREVET EUROPEEN

(45) Date de publication du fascicule du brevet : **12.05.93 Bulletin 93/19**

(51) Int. Cl.$^5$ : **C07D 295/08**, A61K 31/495, C07D 295/10

(21) Numéro de dépôt : **89401640.1**

(22) Date de dépôt : **13.06.89**

(54) **[(Aryl-4-pipérazinyl-1)-2 éthoxy]-3 p-cymène, les dérivés ortho, méta, para monosubstitués ou disubstitués sur le noyau phényle dudit produit, le procédé de préparation desdits dérivés, et les médicaments contenant lesdits composés comme principe actif.**

Jointe à la demande no. 89907194.8/0445116 (numéro de dépôt/numéro de publication de la demande européenne) par décision du 06.12.91.

(30) Priorité : **13.06.88 FR 8807864**

(43) Date de publication de la demande : **20.12.89 Bulletin 89/51**

(45) Mention de la délivrance du brevet : **12.05.93 Bulletin 93/19**

(84) Etats contractants désignés : **AT BE CH DE ES FR GB GR IT LI LU NL SE**

(56) Documents cités :
**EP-A- 0 173 634**
**EP-A- 0 237 411**

(73) Titulaire : **INSTITUT DE RECHERCHES CHIMIQUES ET BIOLOGIQUES APPLIQUEES (I.R.C.E.B.A.) Société à responsabilité limitée dite:
62, Grande-Rue
F-78490 Vicq (FR)**

(72) Inventeur : **Houziaux, Patrick
Chemin des Jonchères
Bazemont F-78580 Maule (FR)**
Inventeur : **Lacolle, Jean-Yves
17 rue Charles de Gaulle
F-78860 Saint Nom la Breteche (FR)**
Inventeur : **Riffaud, Jean-Pierre
108 Avenue des Etats Unis
F-78000 Versailles (FR)**
Inventeur : **Danree, Bernard
53 rue des Grands Champs
F-78300 Poissy (FR)**

(74) Mandataire : **Portal, Gérard et al
Cabinet Beau de Loménie 158, rue de l'Université
F-75340 Paris Cédex 07 (FR)**

EP 0 347 305 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne en tant que produits nouveaux le [(phényl-4-pipérazinyl-1)-2 éthoxy]-3 p-cymène et ses dérivés ortho méta, para monosubstitués ou disubstitués sur le noyau phényl, un procédé de préparation de ces dérivés; elle concerne également les médicaments contenant, en tant que principe actif, au moins un desdits composés.

Les produits chimiques selon l'invention ont pour formule générale :

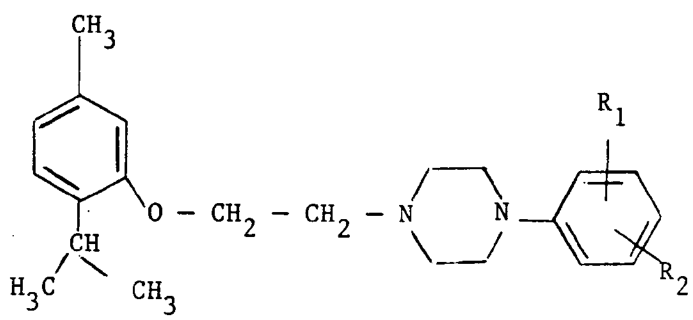

(I)

$R_1$, $R_2$, identiques ou différents, étant choisis parmi un atome d'hydrogène, un atome d'halogène ou un radical hydroxyle, alkyle ayant de 1 à 4 atomes de carbone, alcoxy ayant de 1 à 5 atomes de carbone, phénoxy, benzyloxy, trifluorométhyle, acétyle.

Dans la formule I, les groupes alkyles ou alcoxy peuvent être des groupes à chaîne droite ou ramifiée.

Un groupe alkyle ayant de 1 à 5 atomes de carbone est par exemple un groupe méthyle, éthyle, propyle, isopropyle, butyle, sec-butyle ou tert-butyle, de préférence méthyle.

Un groupe alcoxy ayant de 1 à 5 atomes de carbone est par exemple un groupe méthoxy, éthoxy, propoxy, isopropoxy, butoxy, isobutoxy, néopentoxy, pentoxy, isopentoxy, de préférence éthoxy ou isopropoxy.

Un atome d'halogène est de préférence un atome de chlore.

Les produits selon l'invention peuvent également se présenter sous forme des sels des produits de formule (I) avec un acide pharmaceutiquement acceptable, minéral comme par exemple l'acide chlorhydrique ou l'acide sulfurique ou organique comme par exemple l'acide citrique, tartrique, malique, maléique, fumarique, ou méthane sulfonique.

On connaît déjà les dérivés disubstitués en 3 et en 6 du p-cymène qui présentent des activités cardiovasculaires et antiallergiques. On peut citer en référence les brevets FR-2 570 376 et EP-179009 où le substituant en 3 est un alcoxy substitué par une phénylpipérazine et où le substituant en 6 est un amino hydroxy alcoxy, alors que, dans le brevet EP-173634, le substituant en 6 est un hydroxy.

Dans la présente invention, le p-cymène est substitué seulement en 3 par un alcoxy. C'est donc un dérivé du thymol et de ce fait le composé est original.

Les produits de l'invention sont fabriqués à partir du (chloro-2 éthoxy)3-p-cymène de formule (II) et du (substitué phényl)-1 pipérazine de formule (III) suivant le schéma réactionnel général suivant :

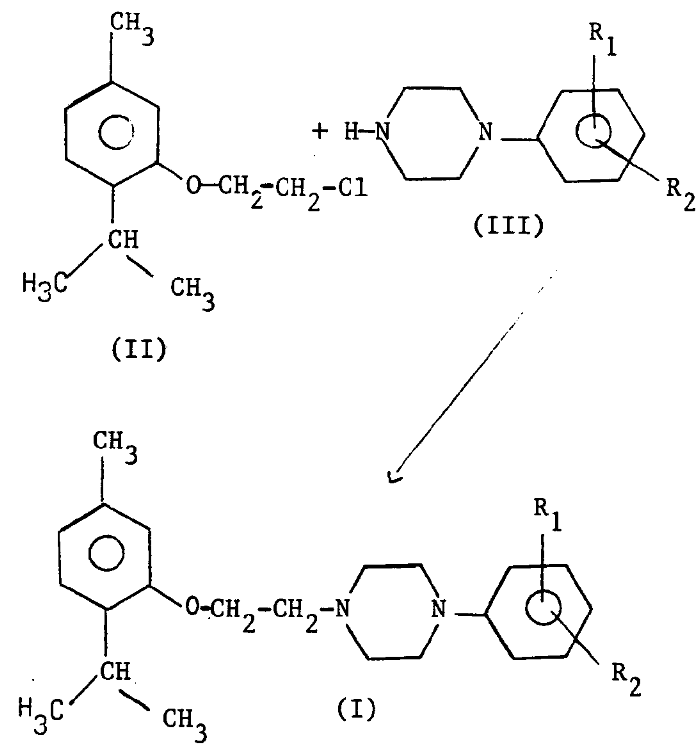

(II) (III) (I)

$R_1$ et $R_2$ étant définis ci-dessus.

Le nombre de moles du composé (III) est à peu près le double du nombre de moles du composé (II).

Les sels pharmaceutiquement acceptables, par exemple les chlorhydrates,sont obtenus de façon connue en mettant en contact une solution du produit de formule (I) avec l'acide considéré (par exemple en faisant barboter de l'acide chlorhydrique dans une solution du produit de formule (I)). De plus, ces sels peuvent comporter une ou deux moles d'acide salifiant par mole de produit de formule (I).

La présente invention concerne également les médicaments caractérisés en ce qu'ils contiennent, comme produit actif, au moins un produit de formule (I); lesdits médicaments peuvent être utiles notamment dans le domaine de l'urologie.

L'invention concerne aussi un procédé de préparation de médicaments notamment utiles dans le domaine de l'urologie, caractérisé en ce qu'on incorpore, comme produit actif au moins un produit de formule I dans un véhicule, excipient ou support pharmaceutiquement acceptable.

Les exemples non limitatifs suivants illustrent les procédés de préparation des produits selon l'invention.

Le test de $AgNO_3$ est un test de pureté du produit.

EXEMPLE 1 :

Synthèse du bichlorhydrate du [(p-fluorophényl-4-pipérazinyl-1)-2 éthoxy]-3 p-cymène.
Nom de code = B 1055. Produit de formule (I) avec $R_1$ = fluoro-4 sous forme de bichlorhydrate.

1. Préparation du [(p-fluorophényl-4 pipérazinyl-1)-2 éthoxy]-3 p-cymène

Dans un tricol de 100 ml, muni d'un réfrigérant, d'une agitation magnétique et d'un thermomètre, on introduit :

21,27 g (0,1 mole) (chloro-2 éthoxy)-3 p-cymène

43,2 g (0,24 mole)p-fluorophényl-1 pipérazine.

Le mélange est chauffé à 140°C durant 10 heures.

Après refroidissement à température ambiante, on verse le milieu réactionnel sur une solution de bicarbonate de sodium à 10%. La phase aqueuse obtenue est extraite deux fois par 100 ml de chlorure de méthylène. Les phases organiques réunies sont lavées par de l'eau saturée en chlorure de sodium. On les sèche ensuite sur sulfate de sodium. Après filtration, le solvant est chassé sous vide. On obtient 48,8 g de produit brut.

Celui-ci a été étêté par distillation fractionnée sous vide (sous azote).

On isole 35,3 g de résidu que l'on recristallise dans 40 ml d'hexane. On obtient ainsi 29,95 g de cristaux beiges (rendement = 84%);$F_{BK}$ = 53-54°C; C.P.V. pureté >99%.

2. Préparation du bichlorhydrate

17,82 g (0,05 mole) de ce produit sont dissous dans l'éther éthylique anhydre. La solution est saturée par un courant d'acide chlorhydrique gazeux sec sur un bain de glace. Les cristaux formés sont filtrés sur verre fritté, lavés par de l'éther éthylique anhydre, puis séchés sous vide sur potasse à 70°C.

On obtient 19,53 g de cristaux beiges (rendement brut = 91%).

Après recristallisation dans l'isopropanol, on isole 16,10 g d'un produit blanc (rendement après recristallisation = 74,9%).

Lesdits cristaux présentent un point de fusion $F_{BK}$ de 157-162°C, des spectres IR et RMN conformes à la structure proposée, un titre $AgNO_3$ de 100%.

EXEMPLE 2

Synthèse du bichlorhydrate du [(phényl-4-pipérazinyl-1)-2 éthoxy]-3 p-cymène. Nom de code = B 1057. Produit de formule (I) avec $R_1$ = $R_2$ = H sous forme de bichlorhydrate.

1. Préparation du [(phényl-4-pipérazinyl-1)-2 éthoxy]-3 p-cymène.

Dans un tricol de 100 ml, muni d'un réfrigérant, d'une agitation magnétique et d'un thermomètre, on introduit :

21,27 g (0,1 mole) (chloro-2-éthoxy)-3 p-cymène

38,93 g (0,24 mole) N-phényl pipérazine.

Le mélange est chauffé à 140°C durant 10 heures.

Après refroidissement à température ambiante, on verse le milieu réactionnel sur une solution de bicarbonate de sodium à 10%.

La phase aqueuse obtenue est extraite deux fois par 100 ml de chlorure de méthylène. Les phases organiques réunies sont lavées par de l'eau saturée en chlorure de sodium. On les sèche ensuite sur sulfate de sodium. Après filtration, le solvant est chassé sous vide. On obtient 44,7 g de produit brut.

Celui-ci est purifié par distillation fractionnée sous vide (sous azote). on isole 32,15 g d'un résidu que l'on recristallise dans un mélange pentane/hexane :3/1. On obtient 29,85 g de cristaux beiges (rendement = 82,2%); $F_{BK}$ = 71-72°C; C.P.V. pureté >99%.

2. Préparation du bichlorhydrate

16,92 g (0,05 mole) de ce produit sont dissous dans l'éther éthylique anhydre. La solution est saturée par un courant d'acide chlorhydrique gazeux sec sur un bain de glace. Les cristaux formés sont filtrés sur verre fritté, lavés par de l'éther éthylique anhydre, puis séchés sous vide sur potasse à 70°C.

On obtient 19,5 g de cristaux beiges (rendement brut = 94,8%).

Après recristallisation dans l'isopropanol, on isole 14,8 g d'un produit blanc (rendement après recristallisation = 72%).

Lesdits cristaux présentent un point de fusion $F_{BK}$ de 162-164°C, des spectres IR et RMN conformes à la structure proposée, un titre $AgNO_3$ de 97,5%. L'analyse élémentaire (formule brute : $C_{22}H_{32}Cl_2N_2O$) a donné les résultats suivants :

| % | Calculé | Trouvé |
|---|---|---|
| C | 63,88 | 64,30 |
| H | 7,86 | 7,84 |
| Cl | 17,14 | 17,36 |
| N | 6,77 | 6,74 |
| O | 4,35 | 4,24 |

EXEMPLE 3 :

Synthèse du monochlorhydrate du [(α,α,α-trifluoro m-tolyl-4 pipérazinyl-1)-2 éthoxy]-3 p-cymène. Nom de code = B 1079. Produit de formule (I) avec $R_1$ = trifluorométhyl-3 sous forme de chlorhydrate.

1. Prépartaion du [(α,α,α-trifluoro m-tolyl-4-pipérazinyl-1)-2 éthoxy]-3 p-cymène.

Dans un tricol de 100 ml, muni d'un réfrigérant, d'une agitation magnétique et d'un thermomètre, on introduit :

21,27 g (0,1 mole) (chloro-2 éthoxy)-3 p-cymène
46,05 g (0,2 mole) N-(α,α,α-trifluoro-m-tolyl)pipérazine.

Le mélange est chauffé à 140°C durant 5 heures.

Après refroidissement à température ambiante, on verse le milieu réactionnel sur une solution de bicarbonate de sodium à 10%. La phase aqueuse obtenue est extraite par 2 fois 100 ml de chlorure de méthylène. Les phases organiques réunies sont lavées par de l'eau saturée en chlorure de sodium. On les sèche ensuite sur sulfate de sodium. Après filtration, le solvant est chassé sous vide. On obtient 56,46 g de produit brut.

Celui-ci est purifié par distillation fractionnée sous vide (sous azote).

On isole 23,62 g d'une huile jaune (rendement = 58,1%).

Le produit obtenu présente un point d'ébullition (sous 0,15 mm Hg) de 195°C, des spectres IR et RMN conformes à la structure proposée.

2. Préparation du chlorhydrate

20,32 g (0,05 mole) de ce produit sont dissous dans un chlorure de méthylène anhydre. La solution est saturée par un courant d'acide chlorhydrique gazeux sec sur un bain de glace. Les cristaux formés sont filtrés sur verre fritté, lavés par de l'éther éthylique anhydre, puis séchés sous vide sur potasse à 70°C.

On obtient 14,48 g de cristaux beiges (rendement brut = 65,4%).

Après recristallisation dans l'éthanol, on isole 5,91 g d'un produit blanc (rendement après recristallisation = 26,7%).

Lesdits cristaux présentent un point de fusion $F_{BK}$ de 146-148°C, des spectres IR et RMN conformes à la structure proposée, un titre $AgNO_3$ de 101%.

EXEMPLE 4 :

Synthèse du chlorhydrate du [(p-acétylphényl-4-pipérazinyl-1)-2 éthoxy]-3 p-cymène. Nom de code=B1105. Produit de formule (I) avec $R_1$ = acétyl-4 sous forme de chlorhydrate.

1. Préparation du[(p-acétylphényl-4 pipérazinyl-1)-2 éthoxy]-3 p-cymène.

Dans un tricol de 100 ml, muni d'un réfrigérant, d'une agitation magnétique et d'un thermomètre, on introduit :

21,27 g (0,1 mole) (chloro-2 éthoxy)-3 p-cymène
40,85 g (0,24 mole) p-pipérazinylacétophénone

Le mélange est chauffé à 145°C durant 1 heure.

Après refroidissement à température ambiante, on verse le milieu réactionnel sur une solution de bicarbonate de sodium à 10%. La phase aqueuse obtenue est extraite deux fois par 100 ml de chlorure de méthylène. Les phases organiques réunies sont lavées par de de l'eau saturée en chlorure de sodium. On les sèche ensuite sur sulfate de sodium. Après filtration, le solvant est chassé sous vide. On obtient 59,3 g de produit brut.

Celui-ci est étêté par distillation fractionnée sous vide (sous azote).

On isole 27,66 g d'un résidu que l'on recristallise dans le mélange I.P.A./Ethanol:5/2.

On obtient ainsi 11,22 g de cristaux beiges (rendement = 29,5%); $F_{BK}$ = 90-92°C; C.P.V. pureté > 99%; Titre perchlorique = 102%; I.R. : conforme à la structure proposée.

2. Préparation du chlorhydrate

9,51 g (0,025 mole) de ce produit sont dissous dans un mélange éther éthylique/chlorure de méthylène anhydre (15/1). La solution est saturée par un courant d'acide chlorhydrique gazeux sec sur un bain de glace. Les cristaux formés sont filtrés sur verre fritté, lavés par de l'éther éthylique anhydre, puis séchés sous vide sur potasse à 70°C.

On obtient 10 g de cristaux beiges (rendement brut = 96%).

Après recristallisation dans le toluène, on isole 7,45 g d'un produit blanc (rendement après recristallisation = 71,5%).

Lesdits cristaux présentent un point de fusion $F_{BK}$ de 150-152°C, des spectres IR et RMN conformes à la structure proposée, un titre $AgNO_3$ de 103,6%.

Exemple 5:

Synthèse du bichlorhydrate du [(p-méthoxyphényl-4 pipérazinyl-1)-2 éthoxy]-3 p-cymène. Nom de code = B 1115. Produit de formule (I) avec $R_1$ = méthoxy-4 sous forme de bichlorhydrate.

1. Préparation du [(p-méthoxyphényl-4 pipérazinyl-1)-2 éthoxy]-3 p-cymène.

Dans un tricol de 100 ml, muni d'un réfrigérant, d'une agitation magnétique et d'un thermomètre, on introduit :

21,27 g (0,1 mole) (chloro-2 éthoxy)-3 p-cymène

38,45 g (0,2 mole) p-méthoxyphényl-1 pipérazine.

Le mélange est chauffé à 140°C durant 10 heures.

Après refroidissement à température ambiante, on verse le milieu réactionnel sur une solution de bicarbonate de sodium à 10%. La phase aqueuse obtenue est extraite deux fois par 100 ml de chlorure de méthylène. Les phases organiques réunies sont lavées par de l'eau saturée en chlorure de sodium. On les sèche ensuite sur sulfate de sodium. Après filtration, le solvant est chassé sous vide. On obtient 56,27 g de produit brut.

Celui-ci est purifié par distillation fractionnée sous vide (sous azote).

On isole 17,43 g d'un liquide jaunâtre (Rendement = 47,3%); $Eb_{0,3\ mm\ Hg}$ = 206-210°C.

2. Préparation du bichlorhydrate

9,2 g (0,025 mole) de ce produit sont dissous dans 100 ml de chlorure de méthylène anhydre. La solution est saturée par un courant d'acide chlorhydrique gazeux sec sur un bain de glace. Les cristaux formés sont filtrés sur verre fritté, lavés par de l'éther éthylique anhydre, puis séchés sous vide sur potasse à 70°C.

On obtient 10,02 g de cristaux bruns (rendement brut = 90,8%).

Après recristallisation dans un mélange éther éthylique/I.P.A. :1/3, on isole 5,36 g d'un produit blanc (rendement après recristallisation = 48,6%).

Lesdits cristaux présentent un point de fusion $F_{BK}$ de 162-164°C, des spectres IR et RMN conformes à la structure proposée, un titre $AgNO_3$ de 98,6%.

EXEMPLE 6 :

Synthèse du chlorhydrate du [(chloro-4 méthyl-2) phényl-4 pipérazinyl-1)-2 éthoxy]-3 p-cymène. Nom de code = B1152. Produit de formule (I) avec $R_1$ = méthyl-2; $R_2$ = chloro-4 sous forme de chlorohydrate.

1. Préparation du [(chloro-4 méthyl-2) phényl-4 pipérazinyl-1)-2 éthoxy]-3 p-cymène.

Dans un tricol de 100 ml, muni d'un réfrigérant, d'une agitation magnétique et d'un thermomètre, on introduit :

21,27 g (0,1 mole) (chloro-2 éthoxy)-3 p-cymène

42,14 g (0,2 mole) (chloro-4 méthyl-2) phényl-1 pipérazine.

Le mélange est chauffé à 140°C durant 5 heures.

Après refroidissement à température ambiante, on verse le milieu réactionnel sur une solution de bicarbonate de sodium à 10%. La phase aqueuse obtenue est extraite 2 fois par 100 ml de chlorure de méthylène. Les phases organiques réunies sont lavées par de l'eau saturée en chlorure de sodium. On les sèche ensuite sur sulfate de sodium. Après filtration, le solvant est chassé sous vide. On obtient 44,60 g de produit brut.

Celui-ci est purifié par distillation fractionnée sous vide (sous azote).

On isole 33,48 g de produit distillé (rendement = 86,5%); $Eb_{0,1\ mm\ Hg}$ = 215-217°C; Titre perchlorique = 100,3%.

2. Préparation du chlorhydrate

19,35 g (0,05 mole) de ce produit sont dissous dans l'éther éthylique anhydre. La solution est saturée par un courant d'acide chlorhydrique gazeux sec sur un bain de glace. Les cristaux formés sont filtrés sur verre fritté, lavés par de l'éther éthylique anhydre, puis séchés sous vide sur potasse à 70°C.

On obtient 23,31 g de cristaux beiges.

Après recristallisation dans le mélange acétate d'éthyle/éthanol : 6/1, on isole 7,11 g d'un produit blanc (rendement après recristallisation = 31,9%).

Lesdits cristaux présentent un point de fusion $F_{BK}$ de 178-180°C, des spectres IR et RMN conformes à la structure proposée, un titre $AgNO_3$ de 100,4%.

EXEMPLE 7 :

Synthèse du bichlorhydrate de l'[(o-méthoxyphényl-4 pipérazinyl-1)-2 éthoxy]-3 p-cymène. Nom de code = B 1168. Produit de formule (I) avec $R_1$ = méthoxy-2 sous forme de bichlorhydrate.

1. Préparation de l'[(o-méthoxyphényl-4 pipérazinyl-1)-2 éthoxy]-3 p-cymène.

Dans un tricol de 100 ml, muni d'un réfrigérant, d'une agitation magnétique et d'un thermomètre, on introduit :

21,27 g (0,1 mole) (chloro-2 éthoxy)-3 p-cymène

38,45 g (0,2 mole) o-méthoxyphényl-1 pipérazine.

Le mélange est chauffé à 100°C durant 10 heures.

Après refroidissement à température ambiante, on verse le milieu réactionnel sur une solution de bicarbonate de sodium à 10%. La phase aqueuse obtenue est extraite 2 fois par 100 ml de chlorure de méthylène. Les phases organiques réunies sont lavées par de l'eau saturée en chlorure de sodium. On les sèche ensuite sur sulfate de sodium. Après filtration, le solvant est chassé sous vide. On obtient 35,49 g de produit brut (Rendement = 96,3%).

2. Préparation du bichlorhydrate

18,43 g (0,05 mole) de ce produit brut sont dissous dans l'éther éthylique anhydre. La solution est saturée par un courant d'acide chlorhydrique gazeux sec sur un bain de glace. Les cristaux formés sont filtrés sur verre fritté, lavés par de l'éther éthylique anhydre, puis séchés sous vide sur potasse à 70°C.

On obtient 21,23 g de cristaux beiges (rendement brut = 96.2%).

Après recristallisation dans le mélange acétate d'éthyle/isopropanol : 3/2, on isole 15,54 g d'un produit blanc (rendement après recristallisation = 70,4%).

Lesdits cristaux présentent un point de fusion $F_{BK}$ de 150-160°C, des spectres IR et RMN conformes à la structure proposée, un titre $AgNO_3$ de 98,2%.

EXEMPLE 8 :

Synthèse du bichlorhydrate de l'[(o-éthoxyphényl-4 pipérazinyl-1)-2 éthoxy]-3 p-cymène. Nom de code = B

1178. Produit de formule (I) avec $R_1$ = éthoxy-2 sous forme de bichlorhydrate.

1. Préparation de l'[(o-éthoxyphényl-4 pipérazinyl-1)-2 éthoxy]-3 p-cymène.

Dans un tricol de 100 ml, muni d'un réfrigérant, d'une agitation magnétique et d'un thermomètre, on introduit :

21,27 g (0,1 mole) (chloro-2-éthoxy)-3 p-cymène
43,32 g (0,21 mole) (éthoxy-2-phényl)-1-pipérazine.

Le mélange est chauffé à 100°C durant 18 heures.

Après refroidissement à température ambiante, on verse le milieu réactionnel sur une solution de bicarbonate de sodium à 10%. La phase aqueuse obtenue est extraite 2 fois par 100 ml de chlorure de méthylène. Les phases organiques réunies sont lavées par de l'eau saturée en chlorure de sodium. On les sèche ensuite sur sulfate de sodium. Après filtration, le solvant est chassé sous vide. On obtient 40,40 g de produit brut.

2. Préparation du bichlorhydrate

22,77 g (0,05 mole) de ce produit brut sont dissous dans l'éther éthylique anhydre. La solution est saturée par un courant d'acide chlorhydrique gazeux sec sur un bain de glace. Les cristaux formés sont filtrés sur verre fritté, lavés par de l'éther éthylique anhydre, puis séchés sous vide sur potasse à 70°C.

On obtient 19,86 g de cristaux beiges (rendement brut = 87,2%).

Après recristallisation dans le mélange acétate d'éthyle/isopropanol : 2/3, on isole 5,87 g d'un produit blanc (rendement après recristallisation = 25,8%).

Lesdits cristaux présentent un point de fusion $F_{BK}$ de 145-150°C, des spectres IR et RMN conformes à la structure proposée, un titre $AgNO_3$ de 97,6%.

L'analyse élémentaire (formule brute : $C_{24}H_{36}Cl_2N_2O_2$) a donné les résultats suivants :

| % | Calculé | Trouvé |
|---|---|---|
| C | 63,14 | 63,14 |
| H | 7,98 | 7,87 |
| Cl | 15,53 | 15,81 |
| N | 6,13 | 6,15 |
| O | 7,22 | 7,34 |

EXEMPLE 9 :

Synthèse du chlorhydrate du [(dichloro-3,4) phényl-4-pipérazinyl-1)-2 éthoxy]-3 p-cymène. Nom de code = B 1184. Produit de formule (I) avec $R_1$ = chloro-3; $R_2$ = chloro-4 sous forme de chlorhydrate.

1. Préparation du [(dichloro-3,4) phényl-4 pipérazinyl-1)-2 éthoxy]-3 p-cymène.

Dans un tricol de 100 ml, muni d'un réfrigérant, d'une agitation magnétique et d'un thermomètre, on introduit :

21,27 g (0,1 mole) (chloro-2 éthoxy)-3 p-cymène
48,53 g (0,21 mole) (dichloro-3,4) phényl-1-pipérazine.

Le mélange est chauffé à 100°C durant 26 heures.

Après refroidissement à température ambiante, on verse le milieu réactionnel sur une solution de bicarbonate de sodium à 10%. La phase aqueuse obtenue est extraite 2 fois par 100 ml de chlorure de méthylène. Les phases organiques réunies sont lavées par de l'eau saturée en chlorure de sodium. On les sèche ensuite sur sulfate de sodium. Après filtration, le solvant est chassé sous vide. On obtient 40,61 g de produit brut.

Celui-ci est purifié par chromatographie sur colonne sous pression sur support de gel de silice (60-200μ).

On isole 30,47 g d'un produit cristallisé (rendement 74,8%).

2. Préparation du chlorhydrate

20,37 g (0,05 mole) de ce produit sont dissous dans l'éther éthylique anhydre. La solution est saturée par un courant d'acide chlorhydrique gazeux sec sur un bain de glace. Les cristaux formés sont filtrés sur verre fritté, lavés par de l'éther éthylique anhydre, puis séchés sous vide sur potasse à 70°C.

On obtient 16,11 g de cristaux beiges (rendement brut = 72,6%).

Après recristallisation dans le mélange acétate d'éthyle/isopropanol : 8/1, on isole 13,89 g d'un produit blanc (rendement après recristallisation = 62,6%).

Lesdits cristaux présentent un point de fusion $F_{BK}$ de 170-172°C, des spectres IR et RMN conformes à la structure proposée, un titre $AgNO_3$ de 95,2%.

EXEMPLE 10

Synthèse du bichlorhydrate du [(p-chlorophényl-4-pipérazinyl-1)-2 éthoxy]-3 p-cymène. Nom de code = B 1191. Produit de formule (I) avec $R_1$ = chloro-4 sous forme de bichlorhydrate.

1. Préparation du [(p-chlorophényl-4 pipérazinyl-1)-2 éthoxy]-3 p-cymène.

Dans un tricol de 100 ml, muni d'un réfrigérant, d'une agitation magnétique et d'un thermomètre, on introduit :

21,27 g (0,1 mole) (chloro-2 éthoxy)-3 p-cymène
41,30 g (0,21 mole) p-chlorophényl-1 pipérazine

Le mélange est chauffé à 100°C durant 28 heures.

Après refroidissement à température ambiante, on verse le milieu réactionnel sur une solution de bicarbonate de sodium à 10%. La phase aqueuse obtenue est extraite 2 fois par 100 ml de chlorure de méthylène. Les phases organiques réunies sont lavées par de l'eau saturée en chlorure de sodium. On les sèche ensuite sur sulfate de sodium. Après filtration, le solvant est chassé sous vide. On obtient 34,01 g de produit brut, (Rendement = 91,2 %).

2. Préparation du bichlorhydrate

18,65 g (0,05 mole) de ce produit sont dissous dans l'éther éthylique anhydre. La solution est saturée par un courant d'acide chlorhydrique gazeux sec sur un bain de glace. Les cristaux formés sont filtrés sur verre fritté, lavés par de l'éther éthylique anhydre, puis séchés sous vide sur potasse à 70°C.

On obtient 22,29 g de cristaux beiges (rendement brut = 100%).

Après recristallisation dans l'éthanol, on isole 14,80 g d'un produit blanc (rendement après recristallisation = 66,4%).

Lesdits cristaux présentent un point de fusion $F_{BK}$ de 165-167°C, des spectres IR et RMN conformes à la structure proposée, un titre $AgNO_3$ de 97,5%.

EXEMPLE 11 :

Synthèse du chlorhydrate du [(m-éthoxyphényl-4-pipérazinyl-1)-2 éthoxy]-3 p-cymène. Nom de code B 1204. Produit de formule (I) avec $R_1$ = éthoxy-3 sous forme de chlorhydrate.

1. Préparation du [(m-éthoxyphényl-4 pipérazinyl-1)-2 éthoxy]-3 p-cymène.

Dans un tricol de 100 ml, muni d'un réfrigérant, d'une agitation magnétique et d'un thermomètre, on introduit :

21,27 g (0,1 mole) (chloro-2-éthoxy)-3 p-cymène
43,32 g (0,21 mole) m-éthoxyphényl-1-pipérazine

Le mélange est chauffé à 100°C durant 33 heures.

Après refroidissement à température ambiante, on verse le milieu réactionnel sur une solution de bicarbonate de sodium à 10%. La phase aqueuse obtenue est extraite 2 fois par 100 ml de chlorure de méthylène. Les phases organiques réunies sont lavées par de l'eau saturée en chlorure de sodium. On les sèche ensuite sur sulfate de sodium. Après filtration, le solvant est chassé sous vide. On obtient 38,40 g de produit brut.

Ce produit est purifié par chromatographie sur colonne sous pression sur support de gel de silice (60-200μ). On isole 32,17 g d'un produit liquide brun (rendement = 84,1%).

2. Préparation du chlorhydrate

19,13 g (0,05 mole) de ce produit sont dissous dans l'éther éthylique anhydre. La solution est saturée par un courant d'acide chlorhydrique gazeux sec sur un bain de glace. Les cristaux formés sont filtrés sur verre fritté, lavés par de l'éther éthylique anhydre, puis séchés sous vide sur potasse à 70°C.

On obtient 19,59 g de cristaux beiges (rendement brut : 93,5%).

Après recristallisation dans l'acétone, on isole 11,88 g d'un produit blanc (rendement après recristallisation = 56,7%).

Lesdits cristaux présentent un point de fusion $F_{BK}$ de 169-170°C, des spectres IR et RMN conformes à la structure proposée, un titre $AgNO_3$ de 109,1%.

EXEMPLE 12

Synthèse du chlorhydrate du [(m-méthoxyphényl-4-pipérazinyl-1)-2 éthoxy]-3 p-cymène. Nom de code = B 1206. Produit de formule (I) avec $R_1$ = méthoxy-3 sous forme de chlorhydrate.

1. Préparation du [(m-méthoxyphényl-4 pipérazinyl-1)-2 éthoxy]-3 p-cymène.

Dans un tricol de 100 ml, muni d'un réfrigérant, d'une agitation magnétique et d'un thermomètre, on introduit :

21,27 g (0,1 mole) (chloro-2 éthoxy)-3 p-cymène
40,37 g (0,21 mole) m-méthoxyphényl-1 pipérazine

Le mélange est chauffé à 100°C durant 25 heures.

Après refroidissement à température ambiante, on verse le milieu réactionnel sur une solution de bicarbonate de sodium à 10%. La phase aqueuse obtenue est extraite 2 fois par 100 ml de chlorure de méthylène. Les phases organiques réunies sont lavées par de l'eau saturée en chlorure de sodium. On les sèche ensuite sur sulfate de sodium. Après filtration, le solvant est chassé sous vide. On obtient 41,20 g de produit brut.

Ce produit est purifié par chromatographie sur colonne sous pression sur support de gel de silice (60-200μ). On isole 28,26 g d'un liquide brun (Rendement = 76,7%).

2. Préparation du chlorhydrate

18,47 g (0,05 mole) de ce produit sont dissous dans l'éthanol. On y ajoute une solution éthanolique d'HCl (renfermant 0,05 mole d'HCl gazeux). Après concentration partielle sous vide, précipitation par de l'éther éthylique anhydre, les cristaux formés sont filtrés sur verre fritté, lavés par de l'éther éthylique anhydre, puis sèchés sous vide sur potasse à 70°C.

On obtient 19,48 g de cristaux beiges (rendement brut = 96,2%).

Après recristallisation dans le mélange isopropanol/éthanol : 4/1, on isole 16,56 g d'un produit blanc (rendement après recristallisation = 81,8%).

Lesdits cristaux présentent un point de fusion $F_{BK}$ de 190-192°C, des spectres IR et RMN conformes à la structure proposée, un titre $AgNO_3$ de 99,6%.

EXEMPLE 13 :

Synthèse du chlorhydrate de l'[(o-méthylphényl-4 pipérazinyl-1)-2 éthoxy]-3 p-cymène. Nom de code= B1213. Produit de formule (I) avec $R_1$ = méthyl-2 sous forme de chlorhydrate.

1. Préparation de l'[(o-méthylphényl-4 pipérazinyl-1)-2 éthoxy]-3 p-cymène.

Dans un tricol de 100 ml, muni d'un réfrigérant, d'une agitation magnétique et d'un thermomètre, on introduit :

21,27 g (0,1 mole) (chloro-2 éthoxy)-3 p-cymène
37,02 g (0,21 mole) o-tolyl -1 pipérazine

Le mélange est chauffé à 100°C durant 18 heures.

Après refroidissement à température ambiante, on verse le milieu réactionnel sur une solution de bicarbonate de sodium à 10%. La phase aqueuse obtenue est extraite 2 fois par 100 ml de chlorure de méthylène. Les phases organiques réunies sont lavées par de l'eau saturée en chlorure de sodium. On les sèche ensuite sur sulfate de sodium. Après filtration, le solvant est chassé sous vide. On obtient 49 g de produit brut.

Ce produit est purifié par chromatographie sur colonne sous pression sur support de gel de silice (60-200μ). On isole 25,52 g d'un liquide brun (Rendement = 72,4%).

2. Préparation du chlorhydrate

17,62 g (0,05 mole) de ce produit sont dissous dans l'éthanol. On y ajoute une solution éthanolique d'HCl (renfermant 0,05 mole d'HCl gazeux). Après concentration partielle sous vide, précipitation par du pentane anhydre, les cristaux formés sont filtrés sur verre fritté, lavés par de l'éthylique anhydre, puis séchés sous vide sur potasse à 70°C.

On obtient 18,67 g de cristaux (rendement brut = 96%).

Après recristallisation dans l'acétate d'éthyle, on isole 16,90 g d'un produit blanc (rendement après recristallisation = 86,9%).

Lesdits cristaux présentent un point de fusion $F_{BK}$ de 158-160°C, des spectres IR et RMN conformes à la structure proposée, un titre $AgNO_3$ de 100,9%.

EXEMPLE 14 :

Synthèse du chlorhydrate du [(méthyl-2 chloro-5 phényl)-4 pipérazinyl-1)-2 éthoxy]-3 p-cymène. Nom de code = B 1256. Produit de formule (I) avec $R_1$ = méthyl-2; $R_2$ = chloro-5 sous forme de chlorhydrate.

1. Préparation du [(méthyl-2 chloro-5 phényl)-4 pipérazinyl-1)-2 éthoxy]-3 p-cymène.

Dans un tricol de 100 ml, muni d'un réfrigérant, d'une agitation magnétique et d'un thermomètre, on introduit :

21,27 g (0,1 mole) (chloro-2 éthoxy)-3 p-cymène
44,25 g (0,21 mole) (méthyl-2 chloro-5) phényl-1-pipérazine

Le mélange est chauffé à 100°C durant 60 heures.

Après refroidissement à température ambiante, on verse le milieu réactionnel sur une solution de bicarbonate de sodium à 10%. La phase aqueuse obtenue est extraite 2 fois par 100 ml de chlorure de méthylène. Les phases organiques réunies sont lavées par de l'eau saturée en chlorure de sodium. On les sèche ensuite sur sulfate de sodium. Après filtration, le solvant est chassé sous vide. On obtient 40,70 g de produit brut.

Ce produit est purifié par distillation fractionnée sous vide (sous azote).

On isole 26 g d'un liquide jaune (rendement = 67,2%); $Eb_{0,15\ mm\ Hg}$ = 225-230°C.

2. Préparation du chlorhydrate

19,35 g (0,05 mole) de ce produit sont dissous dans l'éther éthylique anhydre. La solution est saturée par un courant d'acide chlorhydrique gazeux sec sur un bain de glace. Les cristaux formés sont filtrés sur verre fritté, lavés par de l'éther éthylique anhydre, puis séchés sous vide sur potasse à 70°C.

On obtient 21,17 g de cristaux beiges (rendement brut = 100%).

Après recristallisation dans l'acétate d'éthyle, on isole 8,28 g d'un produit blanc (rendement après recristallisation = 39,1%).

Lesdits cristaux présentent un point de fusion $F_{BK}$ de 166-168°C, des spectres IR et RMN conformes à la structure proposée, un titre $AgNO_3$ de 100,4%.

EXEMPLE 15 :

Synthèse du chlorhydrate de l'[(o-fluorophényl-4 pipérazinyl-1)-2 éthoxy]-3- p-cymène. Nom de code = B 1261. Produit de formule (I) avec $R_1$ = fluoro-2 sous forme de chlorhydrate.

1. Préparation de l'[(o-fluorophényl-4 pipérazinyl-1)-2 éthoxy]-3 p-cymène.

Dans un tricol de 100 ml, muni d'un réfrigérant, d'une agitation magnétique et d'un thermomètre, on introduit :

21,27 g (0,1 mole) (chloro-2 éthoxy)-3 p-cymène
37,85 g (0,21 mole) o-fluorophényl-1 pipérazine

Le mélange est chauffé à 100°C durant 33 heures.

Après refroidissement à température ambiante, on verse le milieu réactionnel sur une solution de bicarbonate de sodium à 10%. La phase aqueuse obtenue est extraite 2 fois par 100 ml de chlorure de méthylène. Les phases organiques réunies sont lavées par de l'eau saturée en chlorure de sodium. On les sèche ensuite sur sulfate de sodium. Après filtration, le solvant est chassé sous vide. On obtient 38,85 g de produit brut.

Ce produit est étêté par distillation fractionnée sous vide (sous azote). Le résidu est repris dans l'éther éthylique, filtré sur papier. Le filtrat est concentré sous vide à sec.

On isole 28,66 g d'une huile brune (rendement = 80,4%).

2. Préparation du chlorhydrate

17,83 g (0,05 mole) de ce produit sont dissous dans l'éther éthylique anhydre. La solution est saturée par un courant d'acide chlorhydrique gazeux sec sur un bain de glace. Les cristaux formés sont filtrés sur verre fritté, lavés par de l'éther éthylique anhydre, puis séchés sous vide sur potasse à 70°C.

On obtient 17,96 g de cristaux grisâtres (rendement = 91,4%).

Après recristallisation dans le mélange pentane/isopropanol : 50/50, on isole 13,16 g d'un produit blanc (rendement après recristallisation = 67%).

Lesdits cristaux présentent un point de fusion $F_{BK}$ de 180-185°C, des spectres IR et RMN conformes à la structure proposée, un titre $AgNO_3$ de 99,4%.

EXEMPLE 16 :

Synthèse du chlorhydrate de l'[(o-chlorophényl-4 pipérazinyl-1)-2 éthoxy]-3 p-cymène. Nom de code = B 1299. Produit de formule (I) avec $R_1$ = chloro-2 sous forme de chlorhydrate.

1. Préparation de l'[(o-chlorophényl-4 pipérazinyl-1)-2 éthoxy]-3 p-cymène.

Dans un tricol de 100 ml, muni d'un réfrigérant, d'une agitation magnétique et d'un thermomètre, on introduit :

21,27 g (0,1 mole) (chloro-2 éthoxy)-3 p-cymène
41,30 g (0,21 mole) o-chlorophényl-1 pipérazine

Le mélange est chauffé à 100°C durant 25 heures.

Après refroidissement à température ambiante, on verse le milieu réactionnel sur une solution de bicarbonate de sodium à 10%. La phase aqueuse obtenue est extraite 2 fois par 100 ml de chlorure de méthylène. Les phases organiques réunies sont lavées par de l'eau saturée en chlorure de sodium. On les sèche ensuite sur sulfate de sodium. Après filtration, le solvant est chassé sous vide. On obtient 38,14 g de produit brut.

Ce produit est purifié par distillation fractionnée sous vide (sous azote).

On isole 23,90 g d'une huile jaune, $Eb_{0,1\ mm\ Hg}$ = 195-197°C (rendement = 64,1%).

2. Préparation du chlorhydrate

18,64 g (0,05 mole) de ce produit sont dissous dans de l'éther éthylique anhydre. La solution est saturée par un courant d'acide chlorhydrique gazeux sec sur un bain de glace. Les cristaux formés sont filtrés sur verre fritté, lavés par de l'éther éthylique anhydre, puis séchés sous vide sur potasse à 70°C.

On obtient 19,55 g de cristaux beiges (rendement = 95.5%).

Après recristallisation dans l'acétate d'éthyle, on isole 14,29 g d'un produit blanc (rendement après recristallisation = 69,8%).

Lesdits cristaux présentent un point de fusion $F_{BK}$ de 136-138°C, des spectres IR et RMN conformes à la structure proposée, un titre $AgNO_3$ de 98,5%.

EXEMPLES 17 à 32 :

En utilisant des processus expérimentaux analogues à ceux décrits dans les exemples 1 à 16, et que l'homme de métier retrouvera facilement, on a préparé d'autres composés dont on a donné au tableau I la formule brute, le poids moléculaire, le point de fusion et le titre $AgNO_3$.

Le tableau suivant est un récapitulatif des exemples de produits de ce brevet.

TABLEAU RECAPITULATIF DES PRODUITS EXEMPLIFIES

FORMULE GENERALE :

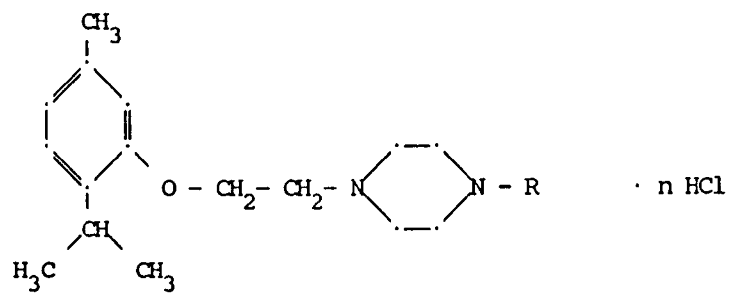

| Exemple | R | n | Code B | Formule brute | P.M. | $F_{BK}$ (°C) | Titre $AgNO_3$ du chlorhydrate |
|---|---|---|---|---|---|---|---|
| 1 | 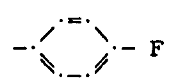 | 2 | 1055 | $C_{22}H_{31}Cl_2FN_2O$ | 429,45 | 157-162 | 100 % |
| 2 | 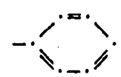 | 2 | 1057 | $C_{22}H_{32}Cl_2N_2O$ | 411,41 | 162-164 | 97,5 % |
| 3 | 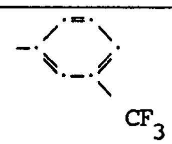 | 1 | 1079 | $C_{23}H_{30}ClF_3N_2O$ | 442,95 | 146-148 | 101 % |
| 4 | 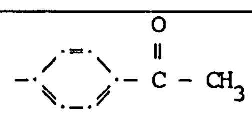 | 1 | 1105 | $C_{24}H_{33}ClN_2O_2$ | 416,98 | 150-152 | 103,6 % |

| Exemple | R | n | Code B | Formule brute | P.M. | $F_{BK}$ (°C) | Titre $AgNo_3$ du chlorhydrate |
|---|---|---|---|---|---|---|---|
| 5 | 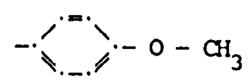 | 2 | 1115 | $C_{23}\ H_{34}\ Cl_2\ N_2\ O_2$ | 441,43 | 162-164 | 98,6 % |
| 6 | 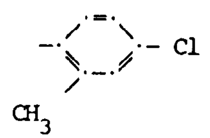 | 1 | 1152 | $C_{23}\ H_{32}\ Cl_2\ N_2\ O$ | 423,42 | 178-180 | 100,4 % |
| 7 | 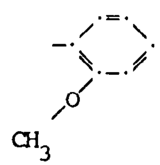 | 2 | 1168 | $C_{23}H_{34}Cl_2\ N_2\ O_2$ | 441,43 | 150-160 | 98,2 % |
| 8 | 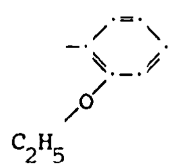 | 2 | 1178 | $C_{24}\ H_{36}\ Cl_2\ N_2\ O_2$ | 455,47 | 145-150 | 97,6 % |
| 9 | 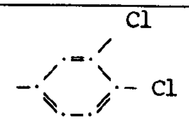 | 1 | 1184 | $C_{22}\ H_{29}\ Cl_3\ N_2\ O$ | 443,83 | 170-172 | 95,2 % |
| 10 | 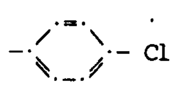 | 2 | 1191 | $C_{22}\ H_{31}\ Cl_3\ N_2\ O$ | 445,85 | 165-167 | 97,5 % |

| Exemple | R | n | Code B | Formule brute | P.M. | $F_{BK}$ (°C) | Titre $AgNO_3$ du chlorhydrate |
|---|---|---|---|---|---|---|---|
| 11 | 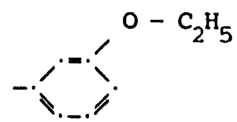 | 1 | 1204 | $C_{24}H_{35}ClN_2O_2$ | 419,0 | 169-170 | 109,1 % |
| 12 | 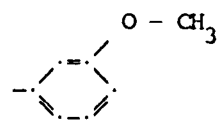 | 1 | 1206 | $C_{23}H_{33}ClN_2O_2$ | 404,97 | 190-192 | 99,6 % |
| 13 | 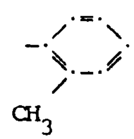 | 1 | 1213 | $C_{23}H_{33}ClN_2O$ | 388,98 | 158-160 | 100,9 % |
| 14 | 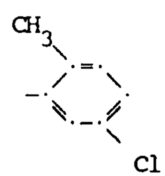 | 1 | 1256 | $C_{23}H_{32}Cl_2N_2O$ | 423,43 | 166-168 | 100,4 % |
| 15 | 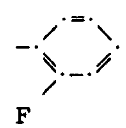 | 1 | 1261 | $C_{22}H_{30}ClFN_2O$ | 392,94 | 180-185 | 99,4 % |
| 16 | 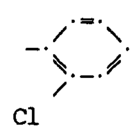 | 1 | 1299 | $C_{22}H_{30}Cl_2N_2O$ | 409,40 | 136-138 | 98,5 % |

| Exemple | R | n | Code B | Formule brute | P.M. | $F_{BK}$ (°C) | Titre $AgNO_3$ du chlorhydrate |
|---|---|---|---|---|---|---|---|
| 17 | 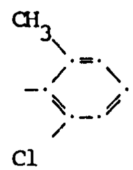 | 1 | 1326 | $C_{23}H_{32}Cl_2N_2O$ | 423,43 | 168-170 | 97,9 % |
| 18 | 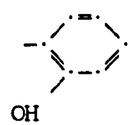 | 1 | 1338 | $C_{22}H_{31}ClN_2O_2$ 0,5 $H_2O$ | 399,95 | 170-172 | 101,6 % |
| 19 | 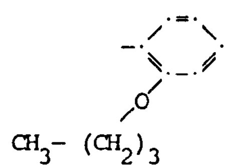 | 1 | 1340 | $C_{26}H_{39}ClN_2O_2$ | 447,07 | 154-156 | 98,6 % |
| 20 | 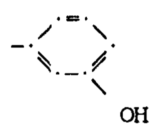 | 1 | 1343 | $C_{22}H_{31}ClN_2O_2$ | 390,95 | 183-184 | 98,6 % |

| Exemple | R | n | Code B | Formule brute | P.M. | $F_{BK}$ (°C) | Titre $AgNO_3$ du chlorhydrate |
|---|---|---|---|---|---|---|---|
| 21 | 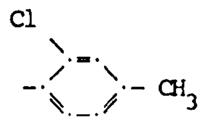 | 1 | 1353 | $C_{23}H_{32}Cl_2N_2O$ | 423,41 | 172-173 | 99 % |
| 22 | 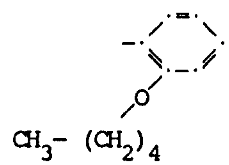 | 1 | 1356 | $C_{27}H_{41}ClN_2O_2$ | 462,0 | 146-148 | 98,3 % |
| 23 | 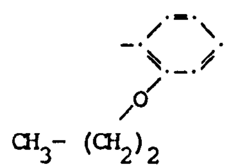 | 1 | 1358 | $C_{25}H_{37}ClN_2O_2$ | 433,04 | 169-171 | 99,3 % |
| 24 | 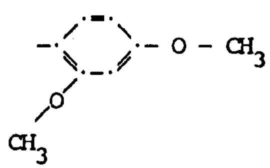 | 1 | 1362 | $C_{24}H_{35}ClN_2O_3$ | 435,00 | 170-172 | 99 % |

| Exemple | R | n | Code B | Formule brute | P.M. | $F_{BK}$ (°C) | Titre $AgNO_3$ du chlorhydrate |
|---|---|---|---|---|---|---|---|
| 25 | 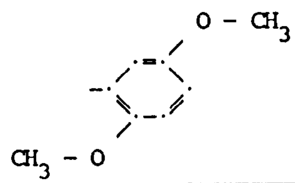 | 1 | 1387 | $C_{24}$ $H_{35}$ Cl $N_2$ $O_3$ | 435,00 | 176-178 | 99,4 % |
| 26 | 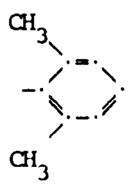 | 1 | 1393 | $C_{24}$ $H_{35}$ Cl $N_2$ O | 403,00 | 174-175 | 100,1 % |
| 27 | 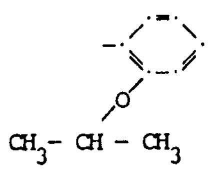 | 1 | 1399 | $C_{25}$ $H_{37}$ Cl $N_2$ $O_2$ | 433,04 | 208-210 | 95,6 % |
| 28 | 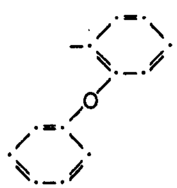 | 1 | 1410 | $C_{28}$ $H_{35}$ Cl $N_2$ $O_2$ | 467,05 | 168-170 | 97 % |

| Exemple | R | n | Code B | Formule brute | P.M. | $F_{BK}$ (°C) | Titre $AgNO_3$ du chlorhydrate |
|---|---|---|---|---|---|---|---|
| 29 | 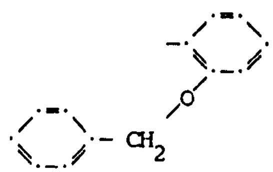 | 1 | 1411 | $C_{29}H_{37}ClN_2O_2$ | 481,08 | 181-182 | 97,4 % |
| 30 | 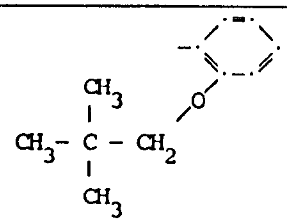 | 1 | 1412 | $C_{27}H_{41}ClN_2O_2$ | 461,09 | 220-222 | 98,5 % |
| 31 | 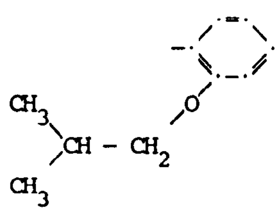 | 1 | 1413 | $C_{26}H_{39}ClN_2O_2$ | 447,06 | 192-194 | 99,2 % |
| 32 | 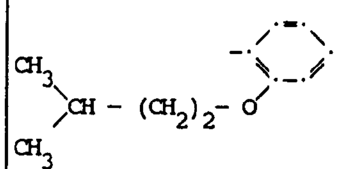 | 1 | 1414 | $C_{27}H_{41}ClN_2O_2$ | 461,09 | 142-144 | 100,2 % |

Les propriétés toxicopharmacologiques des produits faisant l'objet de la présente invention sont décrites ci-après.

1. Toxicité aiguë chez la souris

a) Principe

Les produits ont été administrés par voie orale à dose unique chez la souris. Après une période d'observation de 14 jours, la mortalité a été enregistrée.

Les résultats sont exprimés sous forme de dose létale 50 (D.L.50) en $mg.kg^{-1}$.

b) Résultats.

Ils sont rapportés dans le tableau I.

On peut voir que les produits de l'invention ont une toxicité très faible.

Tableau I : Toxicité aigüe chez la souris.

| PRODUIT | B.1055 | B.1057 | B.1079 | B.1105 | B.1115 | B.1152 | B.1168 | B 1178 |
|---|---|---|---|---|---|---|---|---|
| D.L. 50 mg. $kg^{-1}$ p.o | > 1000 | > 1000 | $\geqslant$ 1000 | $\simeq$ 540 | > 1000 | > 1000 | > 1000 | > 1000 |

| PRODUIT | B.1184 | B.1191 | B.1204 | B.1206 | B.1213 | B.1256 | B.1261 | B 1299 |
|---|---|---|---|---|---|---|---|---|
| D.L. 50 mg. $kg^{-1}$ p.o | > 1000 | > 1000 | > 1000 | > 1000 | > 1000 | > 1000 | > 1000 | > 1000 |

Tableau I : Toxicité aiguë chez la souris.
(suite)

| PRODUIT | B.1326 | B.1338 | B.1340 | B.1343 | B.1353 | B.1356 | B.1358 | B 1362 |
|---|---|---|---|---|---|---|---|---|
| D.L. 50 mg. $kg^{-1}$p.o | > 1000 | > 1000 | > 1000 | > 1000 | > 1000 | > 1000 | > 1000 | 897 |

| PRODUIT | B.1387 | B.1393 | B.1399 |
|---|---|---|---|
| D.L. 50 mg. $kg^{-1}$p.o | > 1000 | > 1000 | ≃ 500 |

2. Détermination de l'activité alpha-bloquante sur canal déférent isolé de rat.

a) Principe.

La stimulation des récepteurs alpha-adrénergiques post-synaptiques par la noradrénaline provoque la contraction du canal déférent isolé. On recherche la concentration de produit en présence de laquelle il faut multiplier par deux la concentration de noradrénaline pour obtenir le même effet qu'en l'absence dudit produit.

Le logarithme changé de signe de cette concentration constitue la valeur de $pA_2$ du produit.

b) Résultats.

Ils sont rapportés dans le tableau II. Ils montrent que les produits se comportent comme des antagonistes compétitifs de la noradrénaline au niveau des récepteurs alpha-adrénergiques, avec une activité alpha-bloquante intéressante.

3. Détermination de l'activité alpha bloquante sur urètre isolé de lapin.

a) Principe

La stimulation des récepteurs alpha-adrénergiques post-synaptiques par la noradrénaline provoque la contraction de l'urètre isolé de lapin.

On recherche la concentration de produit en présence de laquelle l'effet contracturant d'une concentration donnée de noradrénaline, est divisé par deux, par rapport à la contraction induite avant l'addition dudit produit.

Le logarithme changé de signe de cette concentration constitue la valeur de $pD'_2$.

b) Résultats

Certaines valeurs de $pD'_2$ sont rapportées dans le tableau II :

Tableau II : Action alpha-bloquante des produits sur canal déférent isolé de rat.

| PRODUIT | B.1055 | B.1057 | B.1079 | B.1105 | B.1115 | B.1152 | B.1168 | B 1178 |
|---|---|---|---|---|---|---|---|---|
| $pA_2$ | 6,33 | 6,40 | < 6 | 6,27 | 6,54 | N.D. insoluble | 6,71 | 7,14 |

| PRODUIT | B.1184 | B.1191 | B.1204 | B.1206 | B.1213 | B.1256 | B.1261 | B 1299 |
|---|---|---|---|---|---|---|---|---|
| $pA_2$ | N.D. insoluble | 6,48 | 6,00 | 6,77 | 5,96 | < 6 | 6,44 | 6,46 |

N.D. : Non Déterminé

Tableau II : Action alpha-bloquante des produits sur canal déférent isolé de rat.

| PRODUIT | B.1326 | B.1338 | B.1340 | B.1343 | B.1353 | B.1356 | B.1358 | B 1362 |
|---|---|---|---|---|---|---|---|---|
| $pA_2$ | < 6 | 7,39 | < 6 | 6,08 | < 6 | < 6 | < 6 | < 6 |

| PRODUIT | B.1387 | B.1393 | B.1399 |
|---|---|---|---|
| $pA_2$ | 6,45 | < 6 | 6,69* |

*pD'$_2$

4. Détermination de l'activité adrénolytique "in vivo" chez le rat.

a) Principe

L'injection intraveineuse de noradrénaline (0,4 mg. $kg^{-1}$) chez le rat vigile provoque la mort de 100 pour-cent des animaux. L'administration préalable d'une substance à propriété alpha-bloquante permet de réduire cette toxicité.

Les produits revendiqués ont été administrés par voie orale, 30 minutes avant l'injection intraveineuse de noradrénaline.

b) Résultats

Ils sont exprimés sous forme de dose efficace 50 (D.E. 50) dose, en mg.$kg^{-1}$, protégeant 50 pour-cent des animaux.

Les D.E. 50 correspondant aux produits revendiqués sont portées dans le tableau III.

Les composés B.1168, B.1178, B.1299, B.1358, B.1387 et B.1399 exercent une excellente protection contre la toxicité de la noradrénaline, confirmant ainsi l'activité mise en évidence sur organes isolés.

Tableau III : Activité alpha-adrénolytique des composés "in vivo" chez le rat.

| PRODUIT | B.1055 | B.1057 | B.1079 | B.1105 | B.1115 | B.1152 | B.1168 | B 1178 |
|---|---|---|---|---|---|---|---|---|
| D.E. 50 mg. $kg^{-1}$ | > 100 | > 100 | > 100 | 200 | 200 | > 100 | 3 | 2 |

| PRODUIT | B.11184 | B.1191 | B.1204 | B.1206 | B.1213 | B.1256 | B.1261 | B 1299 |
|---|---|---|---|---|---|---|---|---|
| D.E. 50 mg. $kg^{-1}$ | > 100 | > 100 | 56 | 100 | 15 | 121 | 20 | 10 |

Tableau III : Activité alpha-adrénolytique des composés "in vivo" chez le rat.

| PRODUIT | B.1326 | B.1338 | B.1340 | B.1343 | B.1353 | B.1356 | B.1358 | B 1362 |
|---|---|---|---|---|---|---|---|---|
| D.E. 50 mg. $kg^{-1}$ | > 200 | 6,4 | 11,3 | 142 | 30,9 | 31,1 | 2,1 | 37,3 |

| PRODUIT | B.1387 | B.1393 | B.1399 |
|---|---|---|---|
| D.E. 50 mg. $kg^{-1}$ | 6,78 | > 200 | 0,78 |

5. Activité sur l'hyper-pression urétrale chez le lapin

a) Principe.

Une augmentation de la pression artérielle et de la pression urétrale est induite, chez le lapin anesthésié, par une injection intraveineuse de noradrénaline. Ces hyper-pressions peuvent être inhibées par l'administration préalable de molécules à potentiel alpha-bloquant.

L'efficacité est appréciée par la dose inhibitrice 50 (D.I.50) c'est-à-dire la dose inhibant de 50 pourcent les augmentations des pressions artérielle et urétrale. La comparaison des D.I. 50 aux deux niveaux permet de mettre en évidence une éventuelle spécificité urétrale.

b) Résultats

Ils sont présentés dans le tableau IV ci-dessous.

| Produits | D.I. 50 mg. kg$^{-1}$ I.V. | | $R = \frac{Art.}{Uretr.}$ |
|---|---|---|---|
| | Pression artérielle | Pression urétrale | |
| B 1178 | 3,9 | 0,37 | 10,6 |
| B 1338 | 18,98 | 1,6 | 11,8 |
| B 1358 | 5 | 1,22 | 4,1 |
| B 1399 | 1,64 | 0,18 | 9,1 |

TABLEAU IV

Ces résultats montrent l'efficacité plus importante de ces composés au niveau de la pression urétrale qu'au niveau de la pression artérielle.

Des molécules voisines ont déjà été décrites. Elles avaient des propriétés de blocage des récepteurs α-1 et β-adrénergiques au niveau cardiaque et, par conséquent, avaient des propriétés antihypertensives.

Les molécules de la présente invention ont aussi des propriétés de blocage des récepteurs α-1 mais elles sont surtout actives au niveau du bas appareil urinaire et ne conservent qu'une faible action résiduelle au niveau cardiaque.

Les produits conformes à la présente invention présentent donc des propriétés pharmacologiques très intéressantes et leur toxicité est suffisamment faible pour permettre leur utilisation en thérapeutique.

Les produits de l'invention peuvent donc être employés en médecine humaine ou vétérinaire, notamment dans le traitement des dysuries liées à une hypertonie urétrale.

Les produits peuvent être administrés par voie générale (parentérale, orale, rectale) ou par voie topique.

Les compositions pharmaceutiques contenant à titre d'ingrédient actif au moins un produit selon l'invention en combinaison avec un véhicule pharmaceutiquement acceptable peuvent être solides ou liquides et se présenter, par exemple, sous forme de préparations injectables, de comprimés, gélules, granulés. La posologie peut varier dans de larges proportions, en particulier suivant le type et la gravité de l'affection à traiter et suivant le mode d'administration.

Le plus souvent, chez l'adulte, par voie parentérale, elle est comprise entre 0,1 et 0,5 g par jour, par voie orale, elle est comprise entre 0,25 et 4 g par jour.

De telles compositions pharmaceutiques constituent un objet de l'invention. Leur procédé de préparation en constitue un autre.

Il consiste à mélanger à des excipients convenables une dose efficace d'un composé de formule (I).

L'invention concerne enfin l'utilisation des composés de formule (I) pour la préparation de médicaments utiles pour le traitement des dysuries liées à une hypertonie urétrale.

## Revendications

### Revendications pour les Etats contractants suivants : AT, BE, CH, DE, FR, GB, IT, LI, LU, NL, SE

**1.** Produits de formule générale (I) :

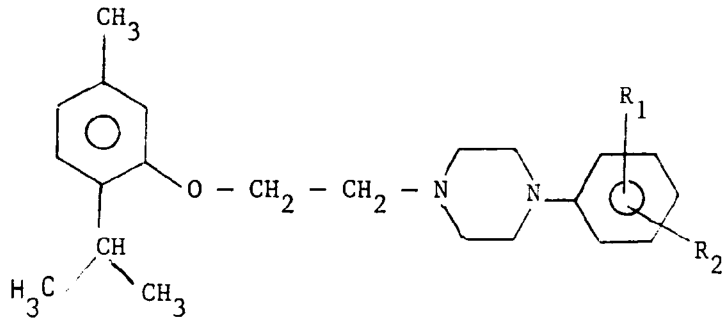

dans laquelle $R_1$, $R_2$, sont identiques ou différents, et sont choisis parmi un atome d'hydrogène, un atome d'halogène ou un radical hydroxyle, alkyle ayant de 1 à 4 atomes de carbone, alcoxy ayant de 1 à 5 atomes de carbone, phénoxy, benzyloxy, trifluorométhyle, acétyle.

2. Méthode de préparation des produits selon la revendication 1, caractérisée en ce que l'on fait réagir le (chloro-2-éthoxy)-3 p-cymène avec la phénylpipérazine de formule générale :

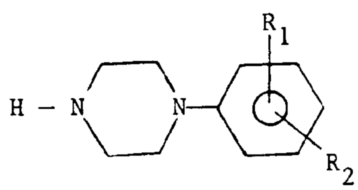

$R_1$ et $R_2$ étant tels que définis à la revendication 1.

3. Nouveaux médicaments utiles en thérapeutique humaine ou vétérinaire contenant une quantité thérapeutique efficace d'au moins un composé selon la revendication 1.

4. Nouveaux médicaments utiles en thérapeutique de l'appareil urinaire, notamment dans le traitement des dysuries liées à une hypertonie urétrale, contenant une quantité thérapeutiquement efficace d'au moins un composé selon la revendication 1 ou 2.

5. Utilisation des composés selon la revendication 1 pour la préparation d'un médicament utile pour le traitement des dysuries liées à une hypertonie urétrale.

**Revendications pour l'Etat contractant suivant : ES**

1. Procédé de préparation des produits de formule générale (I) :

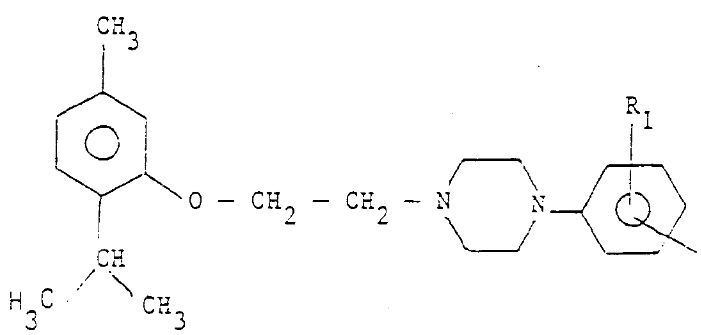

(I)

dans laquelle $R_1$, $R_2$, sont identiques ou différents, et sont choisis parmi un atome d'hydrogène, un atome d'halogène ou un radical hydroxyle, alkyle ayant de 1 à 4 atomes de carbone, alcoxy ayant de 1 à 5 atomes de carbone, phénoxy, benzyloxy, trifluorométhyle, acétyle, caractérisée en ce que l'on fait réagir le (chloro-2-éthoxy)-3-p-cymène avec la phénylpipérazine de formule générale :

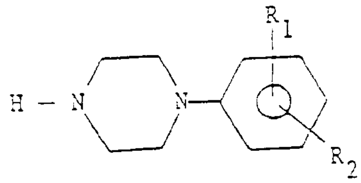

$R_1$ et $R_2$ étant tels que définis précédemment.

2. Procédé de préparation d'une composition pharmaceutique notamment utile pour le traitement des dysuries liées à une hypertonie urétrale, caractérisé en ce qu'il consiste à incorporer dans un excipient pharmaceutiquement acceptable une dose efficace d'un composé de formule générale (I):

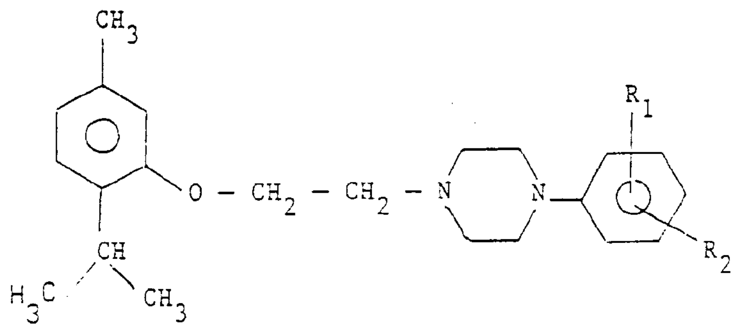

(I)

dans laquelle $R_1$, $R_2$, sont identiques ou différents, et sont choisis parmi un atome d'hydrogène, un atome d'halogène ou un radical hydroxyle, alkyle ayant de 1 à 4 atomes de carbone, alcoxy ayant de 1 à 5 atomes de carbone, phénoxy, benzyloxy, trifluorométhyle, acétyle.

**Revendications pour l'Etat contractant suivant : GR**

1. Produits de formule générale (I) :

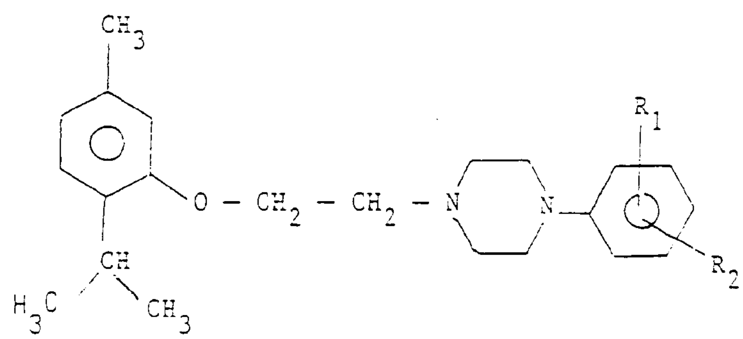

dans laquelle $R_1$, $R_2$, sont identiques ou différents, et sont choisis parmi un atome d'hydrogène, un atome d'halogène ou un radical hydroxyle, alkyle ayant de 1 à 4 atomes de carbone, alcoxy ayant de 1 à 5 atomes de carbone, phénoxy, benzyloxy, trifluorométhyle, acétyle.

2. Méthode de préparation des produits selon la revendication 1, caractérisée en ce que l'on fait réagir le (chloro-2-éthoxy)-3 p-cymène avec la phénylpipérazine de formule générale :

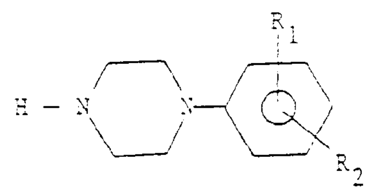

$R_1$ et $R_2$ étant tels que définis à la revendication 1.

3. Utilisation des composés selon la revendication 1 pour la préparation d'un médicament utile pour le traitement des dysuries liées à une hypertonie urétrale.

## Patentansprüche

## Patentansprüche für folgende Vertragsstaaten : AT, BE, CH, DE, FR, GB, IT, LI, LU, NL, SE

1. Produkte der allgemeinen Formel (I):

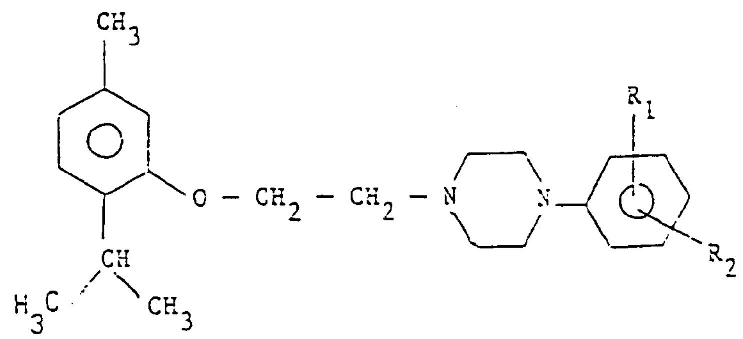

worin $R_1$ und $R_2$, die gleich oder verschieden sein können, ausgewählt werden unter einem Wasserstoffatom, einem Halogenatom, einer Hydroxygruppe, einer Alkylgruppe mit 1 bis 4 Kohlenstoffatomen, einer Alkoxygruppe mit 1 bis 5 Kohlenstoffatomen und einer Phenoxy-, Benzyloxy-, Trifluormethyl- und Acetylgruppe.

2. Verfahren zur Herstellung von Produkten nach Anspruch 1,
dadurch gekennzeichnet, daß
3-(2-Chlorethoxy)-p-cymol mit dem Phenylpiperazin der all- gemeinen Formel

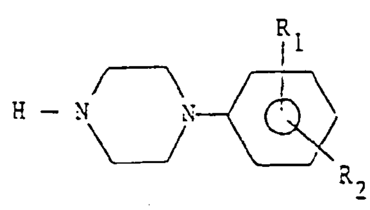

umgesetzt wird, worin $R_1$ und $R_2$ das gleiche bedeuten wie in Anspruch 1.

3. Neue, in der Human- oder Veterinärtherapie verwendbare Medikamente, die eine therapeutisch wirksame Menge von mindestens einer Verbindung nach Anspruch 1 enthalten.

4. Neue, bei der Therapie der Harnwege verwendbare Medikamente, insbesondere zur Behandlung von mit urethraler Hypertonie verbundenen Dysurie, die eine therapeutisch wirksame Menge von mindestens einer Verbindung nach Anspruch 1 oder 2 enthält.

5. Verwendung von Verbindungen nach Anspruch 1 zur Herstellung eines zur Behandlung von mit urethraler

Hypertonie verbundenen Dysurien geeigneten Medikaments.

**Patentansprüche für folgenden Vertragsstaat : ES**

1. Verfahren zur Herstellung von Produkten der allgemeinen Formel (I):

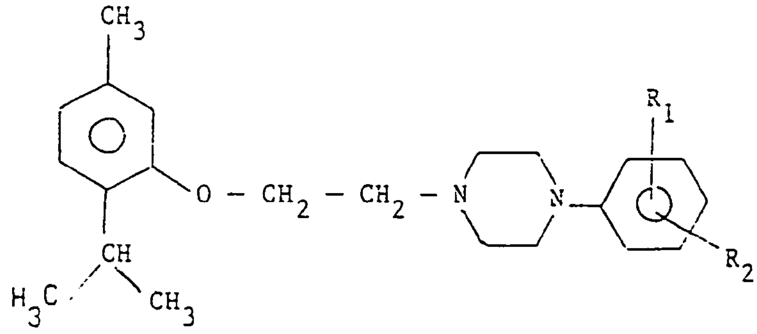

worin $R_1$ und $R_2$, die gleich oder verschieden sein können, ausgewählt werden unter einem Wasserstoffatom, einem Halogenatom oder einer Hydroxygruppe, einer Alkylgruppe mit 1 bis 4 Kohlenstoffatomen, einer Alkoxygruppe mit 1 bis 5 Kohlenstoffatomen und einer Phenoxy-, Benzyloxy-, Trifluormethyl- und Acetylgruppe, dadurch **gekennzeichnet,** daß
3-(2-Chlorethoxy)-p-cymol mit dem Phenylpiperazin der allgemeinen Formel umgesetzt wird:

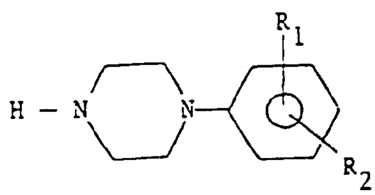

worin $R_1$ und $R_2$ das gleiche bedeuten wie vorher definiert.

2. Verfahren zur Herstellung einer pharmazeutischen Zusammensetzung, die insbesondere zur Behandlung von mit urethraler Hypertonie verbundenen Dysurien geeignet ist, dadurch gekennzeichnet, daß dabei eine wirksame Dosis einer Verbindung der allgemeinen Formel I

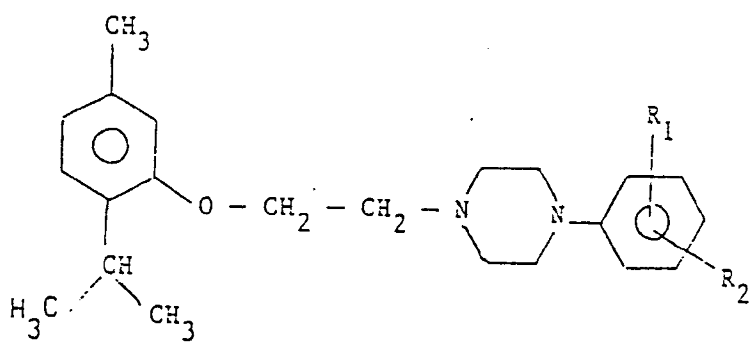

(I)

in einen pharmazeutisch akzeptablen Arzneimittelträger eingebracht wird, worin $R_1$ und $R_2$, die gleich oder verschieden sein können ausgewählt werden unter einem Wasserstoffatom, einem Halogenatom oder einer Hydroxygruppe, einer Alkylgruppe mit 1 bis 4 Kohlenstoffatomen, einer Alkoxygruppe mit 1 bis 5 Kohlenstoffatomen und einer Phenoxy-, Benzyloxy, Trifluormethyl- und Acetylgruppe.

**Patentansprüche für folgenden Vertragsstaat : GR**

1. Produkte der allgemeinen Formel (I):

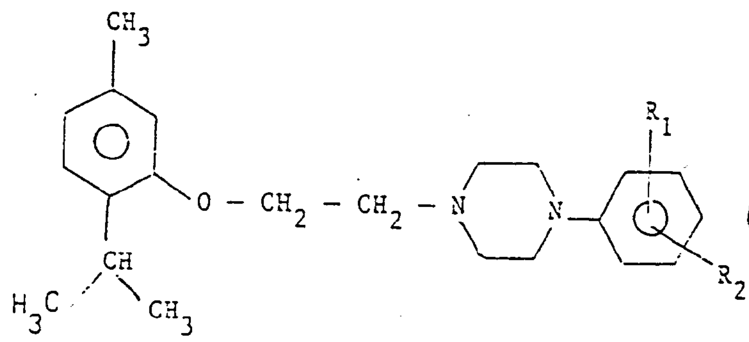 (I)

worin $R_1$ und $R_2$, die gleich oder verschieden sein können, ausgewählt werden unter einem Wasserstoffatom, einem Halogenatom oder einer Hydroxygruppe, einer Alkylgruppe mit 1 bis 4 Kohlenstoffatomen, einer Alkoxygruppe mit 1 bis 5 Kohlenstoffatomen und einer Phenoxy-, Benzyloxy-, Trifluormethyl- und Acetylgruppe.

2. Verfahren zur Herstellung von Produkten nach Anspruch 1, dadurch gekennzeichnet, daß 3-(2-Chlorethoxy)-p-cymol mit Phenylpiperazin der allgemeinen Formel umgesetzt wird:

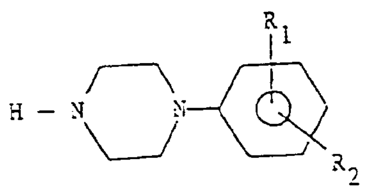

worin $R_1$ und $R_2$ das gleiche bedeuten wie in Anspruch 1.

3. Verwendung von Verbindungen nach Anspruch 1 zur Herstellung eines Medikaments, das zur Behandlung von mit urethraler Hypertonie verbundenen Dysurien geeignet ist.

## Claims

**Claims for the following Contracting States : AT, BE, CH, DE, FR, GB, IT, LI, LU, NL, SE**

1. Products of general formula (I) :

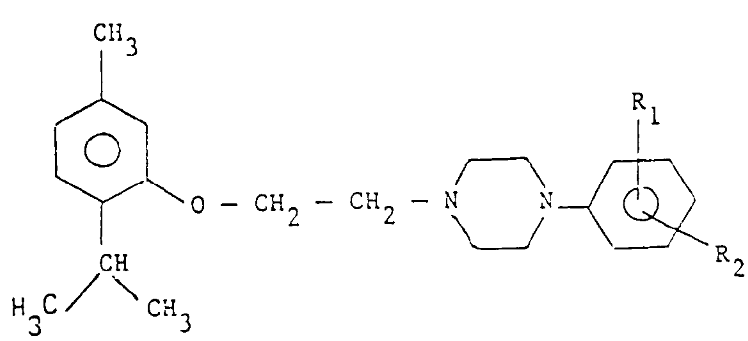

in which $R_1$ and $R_2$ are identical or different and are selected from a hydrogen atom, a halogen atom, a hydroxyl radical, an alkyl radical having from 1 to 4 carbon atoms, an alkoxy radical having from 1 to 5 carbon atoms or a phenoxy, benzyloxy, trifluoromethyl or acetyl radical.

2. Method of preparing the products according to claim 1, characterized in that 3-(2-chloroethoxy)-p-cymene is reacted with the phenylpiperazine of the general formula

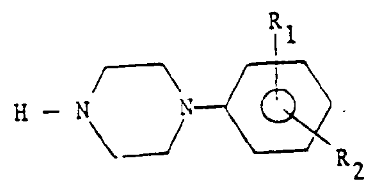

$R_1$ and $R_2$ being as defined in claim 1.

3. Novel drugs useful in human or veterinary therapy, containing a therapeutically effective amount of at least one compound according to claim 1.

4. Novel drugs useful in therapy of the urinary system, especially in the treatment of dysuria associated with ureteral hypertonia, containing a therapeutically effective amount of at least one compound according to claim 1 or 2.

5. Use of the compounds according to claim 1 for the preparation of a drug which is useful for the treatment of dysuria associated with ureteral hypertonia.

**Claims for the following Contrating State : ES**

1. Method of preparing the products of general formula (I) :

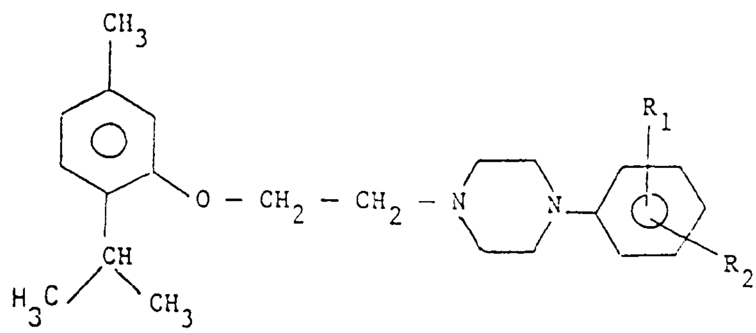

(I)

in which $R_1$ and $R_2$ are identical or different and are selected from a hydrogen atom, a halogen atom, a hydroxyl radical, an alkyl radical having from 1 to 4 carbon atoms, an alkoxy radical having from 1 to 5 carbon atoms or a phenoxy, benzyloxy, trifluoromethyl or acetyl radical, characterized in that 3-(2-chloro-ethoxy)-p-cymene is reacted with the phenylpiperazine of general formula:

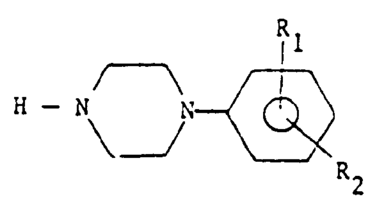

$R_1$ and $R_2$ being as previously defined.

2. Method of preparing a pharmaceutical composition which is especially useful for the treatment of dysuria associated with ureteral hypertonia, characterized in that it- consists in incorporating in a pharmaceutically acceptable excipient an effective amount of a compound of general formula (I):

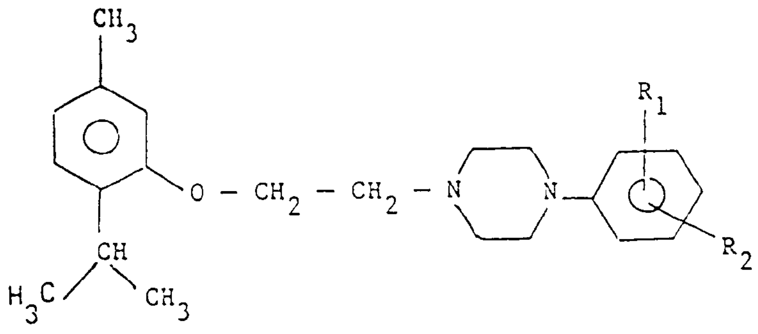

(I)

in which $R_1$ and $R_2$ are identical or different and are selected from a hydrogen atom, a halogen atom, a hydroxyl radical, an alkyl radical having from 1 to 4 carbon atoms, an alkoxy radical having from 1 to 5 carbon atoms or a phenoxy, benzyloxy, trifluoromethyl or acetyl radical.

**Claims for the following Contracting State : GR**

1. Products of general formula (I):

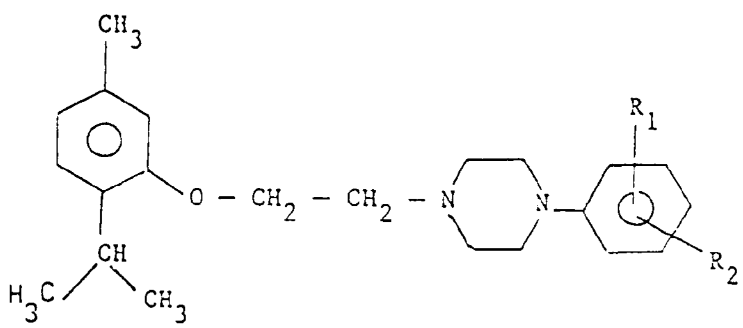

in which $R_1$ and $R_2$ are identical or different and are selected from a hydrogen atom, a halogen atom, a hydroxyl radical, an alkyl radical having from 1 to 4 carbon atoms, an alkoxy radical having from 1 to 5 carbon atoms or a phenoxy, benzyloxy, trifluoromethyl or acetyl radical.

2. Method of preparing products according to claim 1, characterized in that 3-(2-chloroethoxy)-p-cymene is reacted with the phenylpiperazine of the general formula:

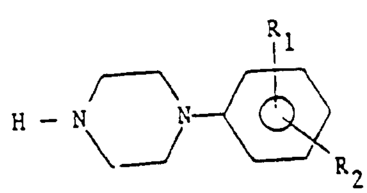

$R_1$ and $R_2$ being as defined in claim 1.

3. Use of the compounds according to claim 1 for the preparation of a drug which is useful for the treatment of dysuria associated with ureteral hypertonia.